# EUROPEAN PATENT SPECIFICATION

(11) **EP 3 047 269 B1**
(45) Date of publication and mention of the grant of the patent: **01.08.2018**
(21) Application number: 14772109.6
(22) Date of filing: 19.09.2014
(51) Int. Cl.: G01N 33/50, G01N 33/53, G01N 33/487

(54) **DIAGNOSIS OF FOETAL DISTRESS**
DIAGNOSE VON FETALEM DISTRESS-SYNDROM
DIAGNOSTIC DE SOUFFRANCE FÉTALE

(30) Priority: 19.09.2013 GB 201316668
(43) Date of publication of application: 27.07.2016
(73) Proprietor: Salurate Limited, Petersfield, Hampshire GU32 3QA (GB)
(72) Inventor: MAGUIRE, Patrick Martin, Co. Wexford (IE)
(74) Representative: Schlich, George
(86) International application number: PCT/EP2014/070053
(87) International publication number: WO 2015/040190

(56) References cited:
- WO-A2-2004/113926
- MANY A ET AL: "HYPERURICEMIA AND XANTHINE OXIDASE IN PREECLAMPSIA, REVISITED", AMERICAN JOURNAL OF OBSTETRICS & GYNECOLOGY, MOSBY, ST LOUIS, MO, US, vol. 174, no. 1, PART 01, 1 January 1996 (1996-01-01), pages 288-291, XP009042145, ISSN: 0002-9378, DOI: 10.1016/S0002-9378(96)70410-6
- CHAPPELL L C ET AL: "A LONGITUDINAL STUDY OF BIOCHEMICAL VARIABLES IN WOMEN AT RISK OF PREECLAMPSIA", AMERICAN JOURNAL OF OBSTETRICS & GYNECOLOGY, MOSBY, ST LOUIS, MO, US, vol. 187, no. 1, 1 July 2002 (2002-07-01), pages 127-136, XP001205032, ISSN: 0002-9378, DOI: 10.1067/MOB.2002.122969
- THANGARATINAM S ET AL: "Accuracy of serum uric acid in predicting complications of pre-eclampsia: a systematic review.", BJOG : AN INTERNATIONAL JOURNAL OF OBSTETRICS AND GYNAECOLOGY APR 2006, vol. 113, no. 4, April 2006 (2006-04), pages 369-378, ISSN: 1470-0328

## Description

The present invention relates to diagnosis or monitoring of foetal distress, in particular distress caused by conditions in the mother other than pre-eclampsia.

Pregnancies are often associated with distress in the foetus. It is important to monitor foetal distress because this may indicate that the pregnancy or labour is compromised. A number of signs of foetal distress have been historically used such as decreased foetal movement felt by the mother, meconium in the amniotic fluid, abnormal indications in cardiotocography and biochemical signs typically assessed by collecting a small sample of baby's blood from a scalp prick through the open cervix during labour.

Foetal distress is thought to be one indicator of a multitude of possible issues in the foetus/pregnancy such as breathing problems, growth disorders, gestational diabetes, a breach position, umbilical cord problems, placental problems, uterine problems and pre-eclampsia.

Treatment of foetal distress depends on its severity, but can range from monitoring as part of a pre-natal care plan to emergency admission for induced labour or hitherto unplanned caesarean section birth.

Pregnancies at risk of developing problems that may cause foetal distress are currently identified by measurements of high gestational blood pressure and/or urinary protein levels. However, these measurements have been reported as being poor predictors of pregnancy problems. As a result a large proportion of apparently high-risk pregnancies, which have been allocated an intensive and expensive care, treatment and diagnostics plan, are trouble-free, and a large proportion of apparently low-risk pregnancies require unexpected medical intervention. Accordingly, current medical practice does not adequately identify pregnancies at risk of developing problems that may cause foetal distress.

Many et al (1996) Am J Obstet Gynecol 174(1 Pt 1):288-91 is a review article that discusses the association between preeclampsia and high serum uric acid concentration and hypothesizes about possible driving forces behind the hyperuricemia.

EP 1634082, originally published as WO 2004/113926, discloses a method for diagnosis of pre-eclampsia in the mother comprising measuring urate in a biological sample from maternal saliva. This enables identification of women at risk of or having pre-eclampsia by the presence of an elevated urate level in maternal saliva.

The method of EP 1634082 has been developed specifically to provide a warning signal for pre-eclampsia and the signal it monitors, salivary urate levels, is subject to regulation according to diurnal rhythms, the stage of pregnancy and the medical/emotional status of the mother.

JP 20062666721 relates to the use of salivary urate for monitoring the health of a subject.

Soukup et al (2012) Diabet. Metabol. Syndrome 4:14, which investigates correlations between salivary urate and cardiometabolic risk factors, suggests that salivary and serum uric acid levels have a linear relationship. Data to support this assertion is not presented. In any case, this study is unrelated to foetal health.

Gao et al (2008) Prenatal Diagnosis 28:810-814 studies whether urate in amniotic fluid is predictive of infant birth outcomes. Sampling of amniotic fluid as used in Gao et al is invasive and thus risks harm to the foetus.

Akahori et al (2012) Gynecol. Obs. Invest. 73:162-167 correlates maternal serum urate with foetal growth and maternal kidney function. Assays were performed at week 36, which is too late to provide any guidance for antenatal care.

Laughton et al (2009) Am. J. Obs. Gynecol. 201:582.e1-582.e6 correlates maternal plasma urate with foetal growth.

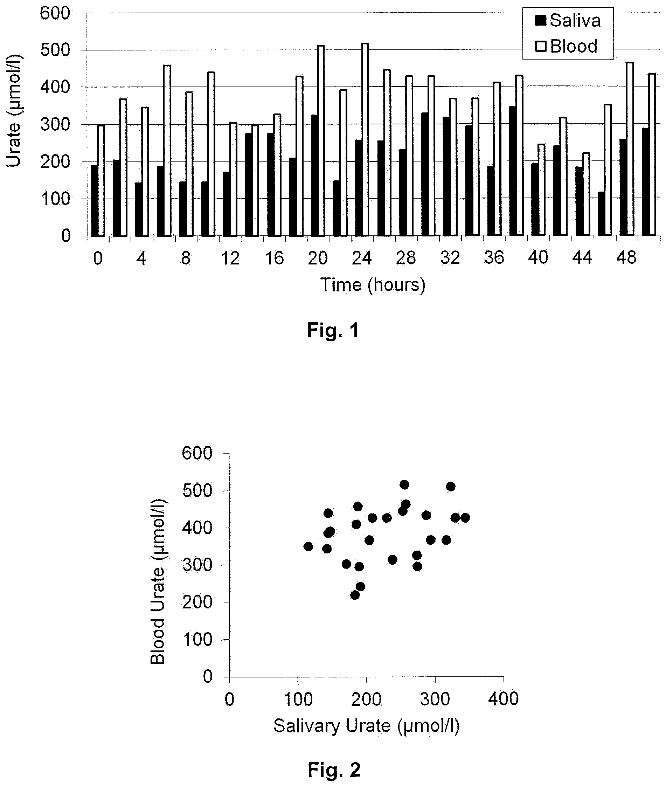

Some known studies are hence based on urate sampling of serum or plasma. The inventors have found, however, that salivary and serum urate levels are uncorrelated (Figures 1 and 2).

The inventors also note that salivary and serum urate are not correlated in conditions such as gout or during/following exercise (Owen-Smith et al (1998) The Lancet 351:1932). Without being bound by any theory, the decoupling of salivary and serum urate may be explained by the salivary urate being sensitive to urate levels in interstitial fluids, which may vary independently from blood urate levels.

There remains a need for an improved or alternative method for diagnosing foetal distress regardless of the cause.

Accordingly, the present invention provides a method of diagnosing and/or monitoring foetal distress comprising measuring uric acid levels in saliva in one or more samples obtained from the expectant mother wherein the distress is other than that caused by pre-eclampsia.

Foetal distress is defined according to the invention as stress in the foetus manifesting in metabolic stress. In making the invention, the inventors have found that metabolic stress in the foetus may be detected in the mother's saliva for example in elevated levels of uric acid, and that this metabolic stress is a warning sign for various specific disorders.

Collection of saliva is not invasive, unlike for example sampling of blood from the mother or the foetus and is not associated with fear for example of pain related to providing a blood sample using a hypodermic needle or finger prick test. Collection of saliva is also more convenient than collection of urine. The level of patient compliance for saliva testing is therefore high, leading to fewer delays in testing and better clinical outcomes. Additionally, collection of saliva does not require the services of a skilled practitioner.

While the diagnosis or monitoring of foetal distress according to the invention does not provide a diagnosis of the specific cause of the distress, it does enable mothers to be allocated or transferred to a more appropriate care, treatment and diagnostics plan.

The method of the invention therefore provides an early warning of potential issues in a pregnancy and the more efficient care of at risk mothers and foetuses. Diagnosis or monitoring can be based upon a single sample, in which case it is preferable to monitor for causes of spikes in urate levels not associated with foetal distress, for example acute stress in the mother. In typical use of the invention, two or more samples, more preferably five or more samples, typically ten or more, or 20 or more samples, are obtained from the expectant mother.

The diagnosis or monitoring can be used to produce a wider ranging diagnosis of pregnancy/foetus difficulties than hitherto available. It is known in the art to provide a test indicative only of pre-eclampsia. The present invention provides diagnosis or monitoring of foetal distress wherein the distress is other than that caused by pre-eclampsia. Thus, for example, a mother known not to be suffering from pre-eclampsia can be tested for foetal distress other than caused by pre-eclampsia. The test of the invention can be carried out with an open mind as to the specific cause of the foetal distress.

Another embodiment of the invention provides a method wherein the mother is not known to have a condition that could cause foetal distress. Thus, the method enables mothers who are otherwise considered to be having a normal pregnancy, and so may not have particular testing done, to be transferred to a more appropriate care, treatment and diagnostics plan.

While the method of the invention may be useful for patients with known past or present issues with pregnancy, it is particularly suitable for mothers who are generally considered to be at low risk of experiencing complications with their pregnancy. Such mothers may be considered to be from around 12 or 16 years old, generally between around 16 and 50 years of age, preferably between around 18 and 40 years of age, and to have a clinically normal blood pressure, and/or a clinically normal level of protein in the urine.

A clinically normal blood pressure according to the invention is defined in the usual manner by clinicians according to local practices, and may comprise a systolic blood pressure of 140 mmHg or less and/or a diastolic blood pressure of 90 mmHg or less.

A clinically normal level of protein according to the invention is defined in the usual manner by clinicians according to local practices, and may comprise less than 300 mg, preferably less than 150 mg of protein in a 24-hour urine sample.

Accordingly, the method of the invention works on the principle that a clinically high uric acid level in the sample indicates foetal distress.

A clinically high uric acid level in saliva according to the invention may be calibrated according to a baseline (normal) level at the beginning of pregnancy, or at another suitable milestone, which baseline may be stable or time/development varied. The normal level may be determined on an individual, group or population level. Typically, a clinically high uric acid level in saliva is above 275 µmol/l, preferably above 350 µmol/l, more preferably above 400 µmol/l. Generally, a pattern of high uric acid levels in the sample indicates foetal distress

The sensitivity of the test can be adjusted by setting the level at which the uric acid level is considered clinically high. There is a trade-off between sensitivity and specificity such that a lower threshold for considering the level as clinically high provides a highly sensitive test, which will have a low specificity (i.e. more false positives) due to detection of normal variations in the levels - e.g. diurnal variations. However, a higher threshold will conversely provide a lower sensitivity with higher specificity.

For diagnosis or monitoring of foetal stress, an embodiment of the invention requires that the uric acid level is clinically high in two or more samples, preferably three or more samples, optionally four or more samples, in which the samples are obtained on different days.

By incorporating a requirement for the detection of repeated high levels of uric acid to trigger a diagnosis or indication of foetal distress, an embodiment of the invention enables an increase in the specificity of the diagnosis by filtering out cases in which fewer than the required number of samples have a high level of uric acid.

In an embodiment of the invention, the sample is obtained once a week. This further enhances patient compliance by establishing a predictable routine.

In another embodiment of the invention, the sample is obtained more frequently than once a week, for example once a day for a period of e.g. 4 days or more, a week or more, two weeks or more, or even longer. The sampling frequency may be varied throughout the monitoring period according to clinical need or interest.

In another embodiment of the invention, the sample is obtained at approximately the same time in the day, which advantageously avoids false positive or false negative measurements e.g. due to natural diurnal rhythms.

Many complications may occur in the late second or third trimester of pregnancies. Accordingly, in an embodiment of the invention, the sample is obtained from week 30, preferably week 20, optionally from week 10 of the pregnancy until birth. Typically, the sample is obtained from week 20 of the pregnancy until birth, optionally at a higher frequency, for example daily, approximately in the period prior to birth. Limiting sampling and testing to particular stages of pregnancy reduces costs, enabling large scale monitoring to be more financially feasible.

The collection of saliva samples from the mother may be carried out cheaply without technically sophisticated equipment, using for example a tube, in which the mother may spit, or an absorbent swab, which may be chewed or simply placed under the tongue. Preferably, the sample is a sublingual salivary sample.

The present invention may be used to detect warning signs for all pregnancy conditions that cause foetal distress, and may indicate one or more of HELLP syndrome, eclampsia, gestational diabetes, gestational hypertension, premature amniorrhexis, placenta accreta and intrauterine growth restriction. Additionally, the present invention may provide an early warning of the possibility of premature or other emergency birth.

Another aspect of the invention provides use of uric acid levels in maternal saliva as an indicator of foetal distress wherein the foetal distress is not caused by pre-eclampsia, in particular distress caused by one or more of HELLP syndrome, eclampsia, gestational diabetes, gestational hypertension, premature amniorrhexis, placenta accreta and intrauterine growth restriction. The indicator is suitable for use in monitoring or diagnostic purposes.

Other optional and preferred embodiments of the use are as described in relation generally to the method of diagnosis or monitoring of the invention.

Specific embodiments of the invention will now be described in the following Examples, with reference to the accompanying drawings, in which:
Fig. 1 shows levels of urate in saliva and blood measured every 2 hours over a 50 hour period; and
Fig. 2 plots the levels of salivary urate against blood urate using the data shown in Figure 1.

### Example 1 - Sample Collection and Uric Acid Measurement

A sample of saliva was collected from a set of pregnant subjects attending antenatal clinic by placing an absorbent swab under the tongue. A drop of saliva was expressed from each swab onto a test strip for a standard hand-held uric acid meter intended for measuring uric acid in whole blood samples. The sample was then tested per the manufacturer's instructions and the measured uric acid level was indicated on the meter and noted.

The indicated uric acid level was calibrated to provide the uric acid concentration from a whole blood sample. Therefore, the salivary uric acid level was calculated from the indicated uric acid level using a correction factor.

The salivary uric acid concentration was determined from samples collected once a week at around the same time of day from week six or week 20 of pregnancy to birth.

### Example 2 - Data Analysis and Diagnosis

Uric acid concentrations were determined according to Example 1 from week six of pregnancy from a random sample of 149 women. These data show that mean salivary uric acid increased non-linearly with gestational age from approximately 100 µmol/l at six weeks to around 250 µmol/l at 40 weeks, the rate of increase rising from week 30 to week 40.

Therefore, the analysis for this Example assumes that salivary uric acid levels of less than 350 µmol/l are normal, levels in the range 350 to 400 µmol/l are marginal, and levels of more than 400 µmol/l are abnormally high.

In 135 subjects, salivary uric acid concentrations were measured according to Example 1 weekly from week 20 of pregnancy until birth and the clinical outcome of each pregnancy noted. The results of this study are set out below.

Outcomes of the study were tabulated into four groups according to the clinical events in the pregnancy as follows:
- Table 1.: Subjects diagnosed as having pre-eclampsia (not claimed)
- Table 2.: Small babies (∼2500g or less) and admissions to the neonatal unit
- Table 3.: Subjects with other medical conditions including diabetes
- Table 4.: Normal pregnancies with abnormal salivary uric acid results

**Table 1 - Subjects diagnosed as having pre-eclampsia**

| | |
|---|---|
| No. of Subjects | 135 |
| Sensitivity of salivary uric acid | 15 subjects were classified as having pre-eclampsia. All 15 showed repeated high salivary uric acid results |
| Diagnostic potential | Number of abnormal salivary uric acid readings per subject diagnosed with pre-eclampsia = 6.6 separate occasions on average of the 15 'positives'. |
| Predictive potential | Average week of first salivary uric acid flag = week 24 |

Referring to Table 1, 15 subjects were classified by the clinicians as having pre-eclampsia. Of these fifteen, ten exhibited gestational hypertension and urinary protein, whilst five showed hypertension only. All fifteen were marked by a pattern of elevated salivary urate levels appearing on six to seven occasions on average. In eleven of the fifteen pre-eclampsia subjects, the first abnormal salivary uric acid reading appeared on average 13 weeks before the first conventional marker (hypertension/proteinuria). The pre-eclampsia salivary uric acid pattern was also exhibited by two subjects who had had severe pre-eclampsia in an earlier pregnancy, but did not show hypertension or urinary protein on this occasion.

There were no false negatives. No subjects showing the 'normal' salivary uric acid pattern were found to have pre-eclampsia or other gestational abnormalities.

**Table 2 - Small babies (∼2500g or less) and admissions to the neonatal unit.**

| Subject No. | Baby weight | Flagged by salivary uric acid | Flagged by CMA | Detected clinical skills | Undiagnosed | Comments |
|---|---|---|---|---|---|---|
| 1 | 2490g | ✔ | ✔ | ✔ | | Previous HELLP; pre-eclampsia emergency caesarean section |
| 49 | 1730g | ✔ | ✔ | ✔ | | HELLP syndrome; admitted to neonatal unit |
| 104 | 1610g | ✔ | ✔ | ✔ | | Severe pre-eclampsia caesarean at 32 weeks; admitted to neonatal unit |
| 37 | 2370g | ✔ | | ✔ | | Premature; ROM; delivered at 34 weeks |
| 45 | 1940g | ✔ | | ✔ | | Fragmented placenta accreta; spontaneous labour at 32 weeks |
| 64 | 2308g | ✔ | | ✔ | | IUGR diagnosed by ultrasound |
| 20 | 2000g | ✔ | | | X | Planned home delivery; foetal distress; emergency admission to neonatal unit |
| 129 | 2540g | ✔ | | | X | Precipitous home delivery at 38 weeks; breathing difficulty; admission to neonatal unit |

| | | | | | | |
|---|---|---|---|---|---|---|
| CMA = Conventional Pre-eclampsia Marker Abnormality (gestational hypertension or proteinuria); HELLP = Haemolysis, Elevated Liver enzymes and Low Platelet count; ROM = rupture of membranes (amniorrhexis); IUGR = intrauterine growth restriction. | | | | | | |

Referring to Table 2, eight subjects delivered small babies. All eight were identifiable by their elevated salivary uric acid patterns. Only three of these exhibited conventional pre-eclampsia marker abnormalities. A further three did not show either hypertension or proteinuria, but were diagnosed by the professional expertise of the ante-natal care team.

Two of the eight subjects were undiagnosed. Both subjects had exhibited elevated salivary uric acid levels starting in week 24. Both were scheduled for home deliveries, and resulted in emergency admissions to the neonatal unit. One of these subjects who had no conventional markers of pre-eclampsia, was scheduled for home delivery but in week 38 required emergency admission to a neonatal unit.

**Table 3 - Miscellaneous conditions showing elevated SalU results.**

| No. of Subjects | Condition |
|---|---|
| 4 | Diabetes (type 2 insulin dependent) |
| 1 | Multiple Sclerosis (in remission) |
| 1 | Pancreatitis |
| 1 | Campylobacter Infection/Diarrhoea |

Referring to Table 3, seven subjects with medical disorders exhibited a pattern of elevated salivary uric acid results. These conditions were known to the obstetrics team prior to delivery.

**Table 4 - Subjects with uneventful outcomes associated with abnormal salivary uric acid readings**

| | |
|---|---|
| No. of SUBJECTS | 11 |
| Frequency of abnormal salivary uric acid readings | Average of 1.5 abnormal readings per subject (compared to 6.6 abnormal readings per subject for pre-eclampsia subjects) |
| Timing of abnormal readings | Average timing of last abnormal reading seen 8.4 weeks before delivery (compared to week of delivery for pre-eclampsia subjects) |

In eleven cases where deliveries were uneventful, the results showed either one or two instances of abnormal salivary uric acid levels prior to week 30, a pattern recognisably less marked than in the cases of gestational abnormality, where elevated salivary uric acid typically occurred on six / seven occasions from week 24 up to delivery.

Therefore, incorporating a requirement of at least two abnormal salivary uric acid readings into the criteria for diagnosing foetal distress reduced the detection of false positives.

### Example 3 - Alternative Data Analysis and Diagnosis

To develop a specific diagnosis method, the data set from Example 2 was reanalysed. Subjects were included for reanalysis if it was the subject's first pregnancy, there was a low clinical risk of pregnancy issues (defined as an absence of information to the contrary in subject's medical records) and the subject was aged 18-40.

The inclusion criteria were formulated to exclude subjects who would already have a known history of pregnancy issues and/or would be selected for a greater level of monitoring by more conventional methods.

Seventy-six subjects were selected according to the stated criteria. Of these, 26 subjects provided two or more saliva samples in which the uric acid level was 275 µmol/l or greater. The clinical outcomes of the 17 of the 26 subjects that exhibited clinical indications are listed in Table 5.

**Table 5 - Clinical outcomes**

| Confirmed Clinical Pre-eclampsia (not claimed) | | Other Clinical Indications | |
|---|---|---|---|
| 9 Subjects | | 8 Subjects | |
| Subject | Severity | Subject | Condition |
| A | 2 | J | Emergency admission at week 39 following planned homebirth. Admission to neonatal unit. Intrauterine growth restriction with a birth weight of 2000g.* |
| B | 1 | | |
| C | 3 | | |
| D | 2 | K | Gestational diabetes. |
| E | 3 | L | Emergency admission at week 34 following planned homebirth. Admission to neonatal unit. Birth weight of 2370g.* |
| F | 1 | | |
| G | 3 | | |
| H | 1 | M | Emergency C. section due to breech position at week 37 Pancreatitis. |
| I | 1 | N | |
| | | O | Intrauterine growth restriction with a birth weight at week 38 was 2308g. |
| | | P | Emergency C. section. Suboptimal cardiotocography. |
| | | Q | Emergency admission at week 38. Admission to neonatal intensive care unit. Intrauterine growth restriction with a birth weight of 2540g. |
| | | | *Not detected by conventional diagnostic indicators |

| | | | |
|---|---|---|---|
| Grading of pre-eclampsia severity was carried out by consultant obstetrician (retired), and codes indicate the following severity: 3 = severe: 2 = moderate; 1=mild. | | | |

There were no false negative results, meaning that no subjects that provided one or no saliva samples in which the uric acid level was 275 µmol/l or greater exhibited clinical indications detected by conventional diagnostic indicators. Thus, the diagnosis detected 100% of subjects in which there were the specified clinical issues with the pregnancies. The method therefore exhibits a higher sensitivity than known current clinical practice, which is reported to have a sensitivity of 46%. There were nine false positive results, meaning that seven subjects provided two or more saliva samples in which the uric acid level was 275 µmol/l or greater, thus indicating apparent foetal distress, yet exhibited no clinical indications detected by conventional diagnostic indicators. Thus, the specificity of the method of the invention is such that a high proportion (85%) of subjects in which there was an indicator of foetal distress had clinical issues with the pregnancies. This level of specificity is higher than current clinical practice, which is reported to have a specificity of 83%.

The method of the invention accurately identified 46 out of 46 negative clinical outcomes.

### Comparative Example 1 - Blood Urate Does Not Predict Salivary Urate

Salivary urate concentrations were determined according to Example 1 and blood urate concentrations were determined using standard methods. The levels of urate in saliva and blood were measured in one subject every 2 hours over a 50 hour period. Both salivary and blood urate varied over the monitoring period (Figure 1). However, an increase or decrease in the salivary urate level was not always associated with a corresponding increase or decrease in blood urate level (Figure 1). A plot of blood urate against salivary urate reveals no useful correlation between the two urate measurements with the data being widely scattered (Figure 2; linear r² = 0.1028). Thus, blood urate did not predict salivary urate.

Accordingly, the invention provides diagnosis of foetal distress other than pre-eclampsia and uses thereof.

## Claims

1. A method of diagnosing and/or monitoring foetal distress comprising measuring uric acid levels in saliva in one or more samples obtained from the expectant mother wherein the distress is other than that caused by pre-eclampsia.

2. A method according to claim 1, wherein the mother is not known to have a condition that could cause foetal distress.

3. A method according to claim 1 or 2, wherein the mother is between 18 and 40 years of age.

4. A method according to any preceding claim, wherein the mother has a) systolic blood pressure of 140mmHg or less and a diastolic blood pressure of 90mmHg or less and/or b) less than 300mg, preferably less than 150mg, of protein in a 24-hour urine sample.

5. A method according to any preceding claim wherein a clinically high uric acid level in the sample indicates foetal distress.

6. A method according to claim 5 wherein the uric acid level is above 275 µmol/l, preferably above 350 µmol/l, more preferably above 400 µmol/l.

7. A method according to claim 5 or claim 6, wherein uric acid level that is high in two or more samples, preferably three or more samples, optionally four or more samples, in which the samples are obtained on different days, indicates foetal distress.

8. A method according to any preceding claim wherein the sample is obtained once a week.

9. A method according to any preceding claim wherein the sample is obtained from week 20 of the pregnancy until birth.

10. A method according to any preceding claim wherein the sample is a sublingual salivary sample.

11. A method according to any preceding claim, wherein the foetal distress is caused by one or more of HELLP syndrome, eclampsia, gestational diabetes, gestational hypertension, premature amniorrhexis, placenta accreta and intrauterine growth restriction.

12. Use of uric acid levels in a maternal saliva sample as an indicator of foetal distress, wherein the foetal distress is not caused by pre-eclampsia

13. Use of uric acid levels in maternal saliva according to claim 12 as an indicator of one or more of HELLP syndrome, eclampsia, gestational diabetes, gestational hypertension, premature amniorrhexis, placenta accreta and intrauterine growth restriction.

## Patentansprüche

1. Verfahren zum Diagnostizieren und/oder Überwachen von fetalem Distress, beinhaltend das Messen der Harnsäurepegel im Speichel in einer oder mehreren Proben, die von einer werdenden Mutter erhalten werden, wobei sich der Distress von dem unterscheidet, der durch Präeklampsie verursacht wird.

2. Verfahren gemäß Anspruch 1, wobei nicht bekannt ist, dass die Mutter einen Zustand aufweist, der fetalen Distress verursachen könnte.

3. Verfahren gemäß Anspruch 1 oder 2, wobei die Mutter zwischen 18 und 40 Jahre alt ist.

4. Verfahren gemäß einem vorhergehenden Anspruch, wobei die Mutter einen a) systolischen Blutdruck von 140 mmHg oder weniger und einen diastolischen Blutdruck von 90 mmHg oder weniger und/oder b) weniger als 300 mg, bevorzugt weniger als 150 mg, Protein in einer 24-Stunden-Harnprobe aufweist.

5. Verfahren gemäß einem vorhergehenden Anspruch, wobei ein klinisch hoher Harnsäurepegel in der Probe fetalen Distress anzeigt.

6. Verfahren gemäß Anspruch 5, wobei der Harnsäurepegel über 275 µmol/l, bevorzugt über 350 µmol/l, bevorzugter über 400 µmol/l liegt.

7. Verfahren gemäß Anspruch 5 oder Anspruch 6, wobei der Harnsäurepegel, der in zwei oder mehreren Proben, bevorzugt drei oder mehreren Proben, optional vier oder mehreren Proben hoch ist, wobei die Proben an unterschiedlichen Tagen erhalten werden, fetalen Distress anzeigt.

8. Verfahren gemäß einem vorhergehenden Anspruch, wobei die Probe einmal pro Woche erhalten wird.

9. Verfahren gemäß einem vorhergehenden Anspruch, wobei die Probe einmal ab Woche 20 der Schwangerschaft bis zur Geburt erhalten wird.

10. Verfahren gemäß einem vorhergehenden Anspruch, wobei die Probe eine sublinguale Speichelprobe ist.

11. Verfahren gemäß einem vorhergehenden Anspruch, wobei der fetale Distress durch eines oder mehrere von HELLP-Syndrom, Eklampsie, Gestationsdiabetes, Gestationshypertonie, vorzeitiger Amniorrhexis, Placenta accreta und intrauteriner Wachstumsretardierung verursacht wird.

12. Verwendung von Harnsäurepegeln in einer mütterlichen Speichelprobe als Indikator für fetalen Distress, wobei der fetale Distress nicht durch Präeklampsie verursacht wird.

13. Verwendung von Harnsäurepegeln im mütterlichen Speichel gemäß Anspruch 12 als Indikator für eines oder mehrere von HELLP-Syndrom, Eklampsie, Gestationsdiabetes, Gestationshypertonie, vorzeitiger Amniorrhexis, Placenta accreta und intrauteriner Wachstumsretardierung.

## Revendications

1. Procédé de diagnostic et/ou de surveillance de la souffrance foetale, comprenant la mesure des niveaux d'acide urique dans la salive dans un ou plusieurs échantillons obtenus de la femme enceinte, la souffrance étant autre que celle causée par la pré-éclampsie.

2. Procédé selon la revendication 1, dans lequel la mère n'a pas d'affection connue pouvant causer la souffrance foetale.

3. Procédé selon la revendication 1 ou 2, dans lequel la mère est âgée de 18 à 40 ans.

4. Procédé selon l'une quelconque des revendications précédentes, dans lequel la mère a a) une tension artérielle systolique de 140 mmHg ou moins et une tension artérielle diastolique de 90 mmHg ou moins et/ou b) moins de 300 mg, de préférence moins de 150 mg, de protéine dans un échantillon d'urine de 24 heures.

5. Procédé selon l'une quelconque des revendications précédentes, dans lequel un niveau d'acide urique cliniquement élevé dans l'échantillon indique une souffrance foetale.

6. Procédé selon la revendication 5, dans lequel le niveau d'acide urique est supérieur à 275 µmol/l, de préférence supérieur à 350 µmol/l, mieux encore supérieur à 400 µmol/l.

7. Procédé selon la revendication 5 ou la revendication 6, dans lequel un niveau d'acide urique qui est élevé dans deux échantillons ou plus, de préférence trois échantillons ou plus, optionnellement quatre échantillons ou plus, dans lequel les échantillons sont obtenus à des jours différents, indique une souffrance foetale.

8. Procédé selon l'une quelconque des revendications précédentes, dans lequel l'échantillon est obtenu une fois par semaine.

9. Procédé selon l'une quelconque des revendications précédentes, dans lequel l'échantillon est obtenu de la semaine 20 de la grossesse jusqu'à la naissance.

10. Procédé selon l'une quelconque des revendications précédentes, dans lequel l'échantillon est un échantillon salivaire sublingual.

11. Procédé selon l'une quelconque des revendications précédentes, dans lequel la souffrance foetale est causée par l'un ou plusieurs de : syndrome HELLP, éclampsie, diabète gestationnel, hypertension gestationnelle, rupture prématurée des membranes, placenta accreta et restriction de croissance intra-utérine.

12. Utilisation des niveaux d'acide urique dans un échantillon de salive maternelle comme indicateur de souffrance foetale, la souffrance foetale n'étant pas causée par pré-éclampsie.

13. Utilisation des niveaux d'acide urique dans la salive maternelle selon la revendication 12, comme indicateur de l'un ou plusieurs de : syndrome HELLP, éclampsie, diabète gestationnel, hypertension gestationnelle, rupture prématurée des membranes, placenta accreta et restriction de croissance intra-utérine.
